# EUROPÄISCHE PATENTSCHRIFT

(11) **EP 3 042 186 B1**
(45) Veröffentlichungstag und Bekanntmachung des Hinweises auf die Patenterteilung: **10.05.2017**
(21) Anmeldenummer: 14758141.7
(22) Anmeldetag: 01.09.2014
(51) Int. Cl.: G01N 25/32, G01N 33/00

(54) **SENSOR ZUR DETEKTION OXIDIERBARER GASE**
SENSOR FOR DETECTING OXIDIZABLE GASES
CAPTEUR DE DÉTECTION DE GAZ OXYDABLES

(30) Priorität: 02.09.2013 DE 102013217465
(43) Veröffentlichungstag der Anmeldung: 13.07.2016
(73) Patentinhaber: Continental Automotive GmbH, 30165 Hannover (DE)
(72) Erfinder: REITMEIER, Willibald, 93155 Hohenschambach (DE); MOOS, Ralf, 95447 Bayreuth (DE); HAGEN, Gunter, 95131 Schwarzenbach am Wald (DE); SCHÖNAUER-KAMIN, Daniela, 95500 Heinersreuth (DE); KITA, Jaroslaw, 95447 Bayreuth (DE); WIEGÄRTNER, Sven, 91278 Pottenstein (DE)
(86) Internationale Anmeldenummer: PCT/EP2014/068495
(87) Internationale Veröffentlichungsnummer: WO 2015/028660

(56) Entgegenhaltungen:
- WO-A1-2010/022285
- DE-A1- 4 317 568
- US-A- 4 063 898
- US-A1- 2005 037 499

## Beschreibung

Die vorliegende Erfindung betrifft einen Sensor zur Detektion oxidierbarer Gase mit den Merkmalen des Oberbegriffs von Patentanspruch 1.

Sensoren zur Detektion oxidierbarer Gase sind bekannt. Sie arbeiten nach dem Exothermie-Prinzip und umfassen zwei Abschnitte zur Temperaturerfassung, von denen einer mit einem katalytisch aktiven Material beschichtet ist. Liegt nun in der Umgebung ein reduzierendes Gas, beispielsweise H₂, C₃H₆, vor (Sauerstoff in der Umgebung vorausgesetzt), wird an der Oberfläche des katalytisch aktiven Materiales das entsprechende Gas oxidieren. Diese exotherme Reaktion lässt die Temperatur dieses Abschnittes (daher einseitig) ansteigen. Aus der Differenztemperaturmessung zwischen der katalytisch aktiven Fläche und der inaktiven Fläche kann dann auf die Gaskonzentration in der Umgebung des Sensors geschlossen werden.

Bei dem hier beschriebenen Sensor des Standes der Technik liegen die beiden vorstehend erwähnten Abschnitte auf einer planaren Fläche vor, und zwar insbesondere auf einer sogenannten Hot-Plate, einer von einem Grundträger thermisch entkoppelten Platte. Zur Erhöhung der Genauigkeit kann zusätzlich ein Heizelement in das Sensorelement integriert werden, um den Sensor bei definierter Temperatur und damit definierter Aktivität des Katalysators zu betreiben und darüber hinaus die Möglichkeit einer Reinigung (thermischer Abbrand von Verunreinigungen/Ablagerungen) zu gewährleisten.

Der vorstehend beschriebene Sensor des Standes der Technik hat einen großen Platzbedarf, da sich die katalytisch aktive Fläche und die inaktive Fläche auf derselben Seite des Sensorelementes bzw. der Hot-Plate befinden. Dieser relativ große Platzbedarf des Sensors ist mit einem erhöhten Energiebedarf verbunden.

Aus der DE 43 17 568 C2 ist ein Sensor mit den Merkmalen des Oberbegriffs von Patentanspruch 1 bekannt. Bei diesem bekannten Sensor befindet sich die katalytisch aktive Fläche auf einem Wärmeübertragungskörper, während die katalytisch inaktive Fläche auf einem anderen Wärmeübertragungskörper angeordnet ist, der sich neben dem einen Wärmeübertragungskörper befindet. Jeweils ein Thermoelement steht mit einem Wärmeübertragungskörper in Verbindung. Die Thermoelemente stehen über eine im Gehäuse des Sensors angeordnete Verbindung miteinander in Kontakt. Auch dieser bekannte Sensor hat daher einen relativ großen Platzbedarf, da die katalytisch aktive Fläche und die katalytisch inaktive Fläche nebeneinander angeordnet sind.

Aus der US 4 063 898 A ist ein Sensor bekannt, der ebenfalls eine katalytisch inaktive Fläche und eine katalytisch aktive Fläche und eine Einrichtung zur Messung der Thermospannung zwischen beiden Flächen aufweist. Auch hier finden zwei nebeneinander angeordnete Thermoelemente Verwendung.

Ein weiterer Sensor zur Detektion oxidierbarer Gase ist aus der US 4 835 108 bekannt. Des Weiteren sind ein katalytischer Gassensor und ein Verfahren zum Herstellen desselben in der DE 39 32 880 A1 beschrieben.

Aus der WO 2010/022285 A1 ist eine Vorrichtung zur Durchführung einer kalorimetrischen Messung bekannt, die eine Micro-Hot-Plate aufweisen kann.

Aus der US 2005/037499 A1 ist ein Messsystem bekannt, das einen Probenhalter, eine Temperaturveränderungsvorrichtung zur Veränderung der Temperatur einer Probe auf dem Probenhalter und eine Messvorrichtung zum Detektieren einer Änderung in der Probe umfasst. Die Messvorrichtung erfasst Änderungen in der Probe, wie beispielsweise Spannungen, Viskosität oder Oberflächenreflexionsvermögen, durch Messung von Änderungen im Streulicht, reflektierten Licht, emittierten Licht oder im elektrischen Widerstand.

Der vorliegenden Erfindung liegt die Aufgabe zugrunde, einen Sensor gemäß Oberbegriff von Patentanspruch 1 zur Verfügung zu stellen, der sich durch einen besonders geringen Platzbedarf und demzufolge einen besonders geringen Energiebedarf auszeichnet.

Diese Aufgabe wird erfindungsgemäß bei einem Sensor der angegebenen Art durch die kennzeichnenden Merkmale von Patentanspruch 1 gelöst.

Erfindungsgemäß werden somit die katalytisch aktive Fläche und die inaktive Fläche auf verschiedenen Seiten einer Hot-Plate angeordnet und über mindestens eine Durchkontaktierung miteinander verbunden. Der Temperaturunterschied zwischen den beiden Flächen wird nunmehr auf direktem Weg (sozusagen durch den Querschnitt des Sensorelementes) gemessen, indem man den Seebeck-Effekt ausnutzt. Der Seebeck-Effekt beruht auf der Ausbildung einer elektrischen Spannung, wenn über einem Testmaterial eine Temperaturdifferenz herrscht. Da im vorliegenden Fall diese Thermospannung zwischen der katalytisch aktiven und der inaktiven Fläche des Sensorelementes gemessen werden soll, ist das Sensorelement mit mindestens einer Durchkontaktierung in diesem Bereich versehen, die als Thermoelement ausgebildet ist. Die entstandene Thermospannung zwischen der katalytisch aktiven Fläche und der inaktive Fläche des Sensorelementes im Bereich der mindestens einen Durchkontaktierung wird mit einer entsprechenden Einrichtung, im einfachsten Fall mit einem Spannungsmessgerät, gemessen. Sie dient als Maß für die entsprechende Temperaturdifferenz, aus der die Gaskonzentration in der Umgebung des Sensors abgeleitet werden kann.

Das Sensorelement umfasst eine Hot-Plate, d. h. eine von einem Grundträger, bei dem es sich beispielsweise um einen entsprechenden Rahmen handeln kann, thermisch entkoppelte Platte. Diese Hot-Plate ist beispielsweise über vier schmale Arme am zugehörigen Rahmen gelagert. Die zur Lagerung verwendeten schmalen Arme stellen eine geringe Wärmeübertragung von der Hot-Plate auf den umgebenden Rahmen sicher.

Erfindungsgemäß weist diese Hot-Plate auf der einen Seite, nämlich der Oberseite oder Unterseite, die katalytisch aktive Fläche und auf der gegenüberliegenden Seite die inaktive Fläche auf, welche über mindestens eine als Thermoelement ausgebildete Durchkontaktierung miteinander verbunden sind.

In Weiterbildung der Erfindung umfasst die mindestens eine Durchkontaktierung ein Material A, stehen beide Seiten der Durchkontaktierung über ein Material B mit einer Vergleichsstelle, an der kein Temperaturgradient vorhanden ist, in Kontakt und stellt das Material B, oder ein Material mit gleichen thermoelektrischen Eigenschaften wie das Material B, eine leitende Verbindung bis zum Anschluss zur Spannungsmessung her. Der Spannungsabgriff erfolgt hierbei an den beiden Zuleitungen aus dem Material B. Die abgegriffene Spannung an den beiden Zuleitungen auf gemeinsamer Vergleichstemperatur ist somit dem Temperaturunterschied ΔT zwischen den beiden Seiten des Sensorelementes bzw. der Hot-Plate proportional und damit auch ein Maß für die Exothermie bzw. Gaskonzentration an der katalytisch beschichteten Fläche.

Bei der vorstehend beschriebenen Ausführungsform sind daher für die Messung der Temperaturdifferenz nach dem beschriebenen Prinzip lediglich zwei Anschlüsse an das Sensorelement (die Hot-Plate) erforderlich. Der Sensor weist daher vorzugsweise lediglich zwei Anschlüsse an das Sensorelement auf.

Den Messeffekt kann man verstärken, indem man für die Durchkontaktierung (Material A) gezielt Materialien mit hohem Seebeck-Koeffizienten verwendet. Bei dem erfindungsgemäß ausgebildeten Sensor ist daher das Material A der mindestens einen Durchkontaktierung vorzugsweise ein Material mit einem hohen Seebeck-Koeffizienten.

Die Vergleichsstelle befindet sich vorzugsweise am Rahmen der Hot-Plate. Der Rahmen und die Arme bestehen vorzugsweise aus Keramikmaterial. Fährt man mit zwei gleichen Drähten (z. B. aus Kupfer) oder Dickschichtleiterbahnen weiter zur Elektronik, so wird nur die Thermospannung, die dem ΔT der Hot-Plate-Seiten entspricht, gemessen.

Bei einer weiteren Ausführungsform umfasst der Sensor eine Reihenschaltung von Thermopaaren über die Fläche des Sensorelementes. Auf diese Weise kann der Spannungswert für die Temperaturdifferenz vergrößert werden, so dass sich insgesamt ein verstärkter Effekt ergibt. Erfindungsgemäß kann dies dadurch bewerkstelligt werden, dass der Sensor mehrere in Reihe geschaltete Durchkontaktierungen aus unterschiedlichen Materialien im Sensorelement bzw. in der Hot-Plate aufweist. Hierbei bestehen die in Reihe geschalteten Durchkontaktierungen vorzugsweise abwechselnd aus Materialien A und B, die voneinander abweichende Seebeck-Koeffizienten besitzen. Hierbei kann beispielsweise ein Material (z. B. Material A) ein n-Leiter und das andere Material der Durchkontaktierung in der Hot-Plate ein p-Leiter sein. Man kann aber dafür auch Material B verwenden. Generell muss der Seebeck-Koeffizient (die Thermokraft) der Materialien A und B voneinander abweichen.

Neben Metallen sind als Materialien für die Durchkontaktierung keramische Leiter oder Halbleiter geeignet, da diese üblicherweise eine höhere Thermokraft (einen größeren Seebeck-Koeffizienten) besitzen und nicht so gut thermisch leitfähig sind. Damit sind die beiden Seiten des Sensorelementes bzw. der Hot-Plate besser entkoppelt. Die mindestens eine Durchkontaktierung im Sensorelement ist daher vorzugsweise als keramischer Leiter ausgebildet.

Die mindestens eine Durchkontaktierung im Sensorelement bzw. in der Hot-Plate braucht nicht komplett verfüllt zu sein. Vielmehr tritt eine wesentlich bessere Entkopplung auf, wenn nur die Ränder der Durchkontaktierung mit dem Leiter verfüllt sind. Bei dem erfindungsgemäß ausgebildeten Sensor sind daher vorzugweise lediglich die Randbereiche der mindestens einen Durchkontaktierung im Sensorelement mit einem Leiter verfüllt.

Des Weiteren kann auch ein Dickschicht-Mäander im Sensorelement bzw. in der Hot-Plate "vergraben" werden, um weitere Funktionalitäten, wie eine Beheizung auf definierte Temperatur und/oder die Messung der Absoluttemperatur, zu integrieren. Durch die definierte Temperatur und den Leistungsbedarf, diese zu halten, lassen sich weitere Aussagen treffen.

Als Aufbautechnologie für das Sensorelement wird vorzugsweise die keramische Mehrlagentechnologie (LTCC/HTCC) vorgeschlagen. Die geringe Wärmeleitfähigkeit solcher Trägermaterialien unterstützt die Separation der beiden Temperaturniveaus. Gut geeignet sind Gläser und Glas enthaltende Verbindungen, die auch als LTCC-Tapes erhältlich sind, denn hier ist die Wärmeleitfähigkeit noch kleiner als bei den klassischen LTCC-Keramiken. Des Weiteren bietet die LTCC-Technologie die Möglichkeit kostengünstiger Herstellung, frei wählbare Geometrien, mehrlagige Strukturen und Robustheit für harsche Umgebungsbedingungen.

Zur Auswertung der Signale bieten sich verschiedene Beschaltungen an. Als besonders günstig erweist sich die Brückenschaltung. Bei dieser können bei entsprechender Platzierung der Widerstände an der Vergleichsstelle auch gleich die absoluten Temperaturschwankungen des Abgases mit kompensiert werden.

Der erfindungsgemäß ausgebildete Sensor basiert daher auf einer Kombination des Seebeck-Effektes mit einem thermischen Gassensor, die insbesondere bei einer Hot-Plate realisiert ist. Es ergeben sich zusätzliche Auswertemöglichkeiten, eine kompakte Baugröße (Nutzung von Vorder- und Rückseite), ein besonders geringer Leistungsbedarf. Weitere Temperaturmessfühler sind nicht erforderlich. Ein weiterer Vorteil besteht darin, dass weniger Leitungsanschlüsse benötigt werden. Mit einer Reihenschaltung (Thermosäule) kann das Differenzsignal verstärkt werden, ebenfalls durch die Wahl eines "kühlen" Referenzmesspunktes.

Vorzugsweise wird eine ΔT-Messung und absolute T-Messung über die entsprechende Beschaltung durchgeführt.

Die Durchkontaktierung ist vorzugsweise nur am Rand metallisiert, idealerweise aber nicht anliegend. Die katalytisch nichtaktive Seite ist vorzugsweise thermisch gut an den Träger angekoppelt, während dies bei der katalytisch aktiven Seite vorzugsweise nicht der Fall ist. Das Material A und/ oder B kann zusätzlich mit katalytisch aktivem Material abgedeckt oder verunreinigt sein oder sich auch in einer Zwischenschicht befinden.

Die vorstehend erwähnte Ausführung in LTCC/HTCC besitzt Vorteile in Bezug auf ihre thermischen Eigenschaften sowie ihren Aufbau.

Die Erfindung wird nachfolgend anhand von Ausführungsbeispielen in Verbindung mit der Zeichnung im Einzelnen erläutert. Es zeigen:
- Figur 1: eine schematische Draufsicht auf ein als Hot-Plate ausgebildetes Sensorelement;
- Figur 2: einen Schnitt durch das Sensorelement der Figur 1;
- Figur 3: einen Schnitt durch eine weitere Ausführungsform eines Sensorelementes; und
- Figur 4: einen Schnitt durch noch eine weitere Ausführungsform eines Sensorelementes.

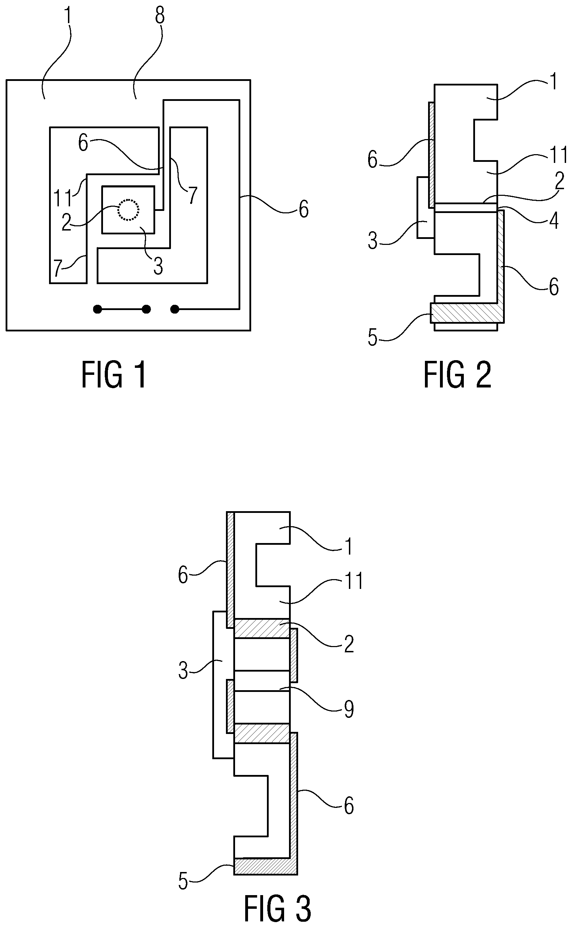

Die Figuren 1 und 2 zeigen schematisch ein Sensorelement 1, das eine sogenannte Hot-Plate 11 umfasst, welche über zwei schmale Arme 7 an einem Rahmen 8 aufgehängt ist. Auf der einen Seite der Hot-Plate 11, hier der Oberseite, befindet sich eine katalytisch aktive Fläche 3, während auf der gegenüberliegenden Seite eine katalytisch inaktive Fläche 4 vorgesehen ist. Beide Flächen sind über eine Durchkontaktierung 2, die als Thermoelement ausgeführt ist, miteinander verbunden.

Das hier dargestellte Sensorelement 1 ist Teil eines Sensors, der zum Detektieren oxidierbarer Gase nach dem Exothermie-Prinzip ausgebildet ist. Insbesondere dient der Sensor zur Kohlenwasserstoff-Detektion (HC-Detektion), Kohlenmonoxid-Detektion (CO-Detektion) oder Wasserstoff-Detektion (H2-Detektion). Wenn in der Umgebung des Sensorelementes 1 ein reduzierendes Gas vorliegt, wobei Sauerstoff in der Umgebung vorausgesetzt wird, wird das Gas an der Oberfläche der katalytisch aktiven Fläche 3 oxidiert. Diese exotherme Reaktion lässt die Temperatur auf dieser Fläche ansteigen, während die Temperatur der katalytisch inaktiven Fläche 4 nicht ansteigt. Der Temperaturunterschied zwischen diesen beiden Flächen 3 und 4 wird nunmehr auf direktem Weg durch den Querschnitt der Hot-Plate 11 gemessen, indem man den Seebeck-Effekt ausnutzt. Mit anderen Worten, durch die entsprechende Temperaturdifferenz wird an der als Thermoelement ausgebildeten Durchkontaktierung 2 eine Thermospannung erzeugt, die gemessen und als Maß für die Temperaturdifferenz und damit die Gaskonzentration in der Umgebung des Sensors ausgewertet wird.

Bei der hier dargestellten Ausführungsform besteht die Durchkontaktierung 2 aus einem Material A. Beide Seiten der Durchkontaktierung 2 stehen über ein Material B mit einer Vergleichsstelle in Kontakt, an der kein Temperaturgradient herrschen sollte. In den Figuren 1 und 2 ist dies mit den Leitungen 6 und der Durchkontaktierung 5 (Vergleichsstelle) dargestellt. Die entsprechenden Leitungen 6 aus dem Material B stellen die leitende Verbindung bis zum Anschluss zur Spannungsmessung (nicht gezeigt) her. Der Spannungsabgriff U an den beiden Leitungen (Zuleitungen) 6 auf gemeinsamer Vergleichstemperatur ist so mit dem Temperaturunterschied ΔT zwischen den beiden Seiten der Hot-Plate proportional und damit auch ein Maß für die Exothermie bzw. Gaskonzentration an der katalytisch aktiven Fläche 3.

Figur 3 zeigt eine Ausführungsform eines Sensorelementes 1, die eine Reihenschaltung von Thermopaaren über die Fläche der Hot-Plate aufweist. Hierbei sind mehrere Durchkontaktierungen 2, 9 in der Hot-Plate vorhanden, wobei die Durchkontaktierungen 2, 9 abwechselnd aus unterschiedlichen Materialien bestehen. Der Seebeck-Koeffizient (die Thermokraft) des Materials A der Durchkontaktierung 2 weicht hierbei vom Seebeck-Koeffizienten der Durchkontaktierungen 9 ab. Im Übrigen entspricht das Konstruktions- und Messprinzip der Ausführungsformen der Figuren 1 und 2.

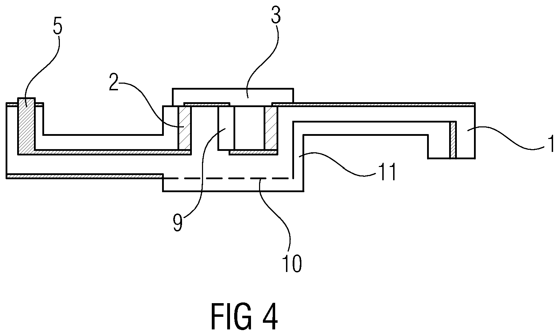

Figur 4 zeigt eine Ausführungsform eines Sensorelementes 1, bei der zusätzlich in die Hot-Plate eine Heizung 10 integriert ist. Hiermit ist eine Beheizung der Hot-Plate 11 auf eine definierte Temperatur möglich.

## Patentansprüche

1. Sensor zur Detektion oxidierbarer Gase mit einem Sensorelement, das eine katalytisch inaktive Fläche und eine katalytisch aktive Fläche aufweist, und mit einer Einrichtung zur Ermittlung der Temperaturdifferenz zwischen den beiden Flächen zur Bestimmung der Gaskonzentration in der Umgebung des Sensors auf der Basis der ermittelten Temperaturdifferenz, wobei sich die katalytisch aktive Fläche auf der einen Seite und die katalytisch inaktive Fläche auf der gegenüberliegenden Seite des Sensorelementes befinden und beide Flächen über ein Thermoelement miteinander verbunden sind und wobei der Sensor eine Einrichtung zur Messung der Thermospannung zwischen der katalytisch aktiven Fläche und der inaktiven Fläche des Sensorelementes als Maß für die Temperaturdifferenz und damit die Gaskonzentration umfasst, **dadurch gekennzeichnet, dass** das Sensorelement (1) eine über schmale Arme (7) an einem Grundträger (8) gelagerte Hot-Plate (11) umfasst, auf deren Vorderseite die katalytisch aktive Fläche (3) und auf deren Rückseite die katalytisch inaktive Fläche (4) oder umgekehrt ausgebildet sind, und dass das Thermoelement von mindestens einer sich durch die Hot-Plate (11) erstreckenden und die beiden Flächen (3, 4) miteinander verbindenden Durchkontaktierung (2, 9) gebildet ist, in deren Bereich die Thermospannung gemessen wird.

2. Sensor nach Anspruch 1, **dadurch gekennzeichnet, dass** die mindestens eine Durchkontaktierung (2) ein Material A umfasst, dass beide Seiten der Durchkontaktierung (2) über ein Material B mit einer Vergleichsstelle, an der kein Temperaturgradient vorhanden ist, in Kontakt stehen und dass das Material B eine leitende Verbindung bis zum Anschluss zur Spannungsmessung herstellt.

3. Sensor nach Anspruch 2, **dadurch gekennzeichnet, dass** der Spannungsabgriff an den beiden Zuleitungen (6) aus dem Material B erfolgt.

4. Sensor nach einem der vorangehenden Ansprüche, **da- durch gekennzeichnet**, dass er lediglich zwei Anschlüsse an das Sensorelement (1) aufweist.

5. Sensor nach einem der vorangehenden Ansprüche, **dadurch gekennzeichnet, dass** das Material A der mindestens einen Durchkontaktierung (2) ein Material mit einem vom Betrag her hohen Seebeck-Koeffizienten ist.

6. Sensor nach einem der Ansprüche 2 bis 5, **dadurch gekennzeichnet, dass** sich die Vergleichsstelle an einem Rahmen (8) der Hot-Plate (11) befindet.

7. Sensor nach einem der vorangehenden Ansprüche, **dadurch gekennzeichnet, dass** er eine Reihenschaltung von Thermopaaren über die Fläche des Sensorelementes (1) umfasst.

8. Sensor nach Anspruch 7, **dadurch gekennzeichnet, dass** er mehrere in Reihe geschaltete Durchkontaktierungen (2, 9) aus unterschiedlichen Materialien im Sensorelement (1) aufweist.

9. Sensor nach Anspruch 8, **dadurch gekennzeichnet, dass** die in Reihe geschalteten Durchkontaktierungen (2, 9) abwechselnd aus Materialien A und B bestehen, die voneinander abweichende Seebeck-Koeffizienten besitzen.

10. Sensor nach einem der vorangehenden Ansprüche, **dadurch gekennzeichnet, dass** die mindestens eine Durchkontaktierung (2, 9) im Sensorelement (1) als keramischer Leiter ausgebildet ist.

11. Sensor nach einem der vorangehenden Ansprüche, **dadurch gekennzeichnet, dass** lediglich die Randbereiche der mindestens einen Durchkontaktierung (2, 9) im Sensorelement (1) mit einem Leiter verfüllt sind.

## Claims

1. Sensor for detecting oxidizable gases, having a sensor element which has a catalytically inactive surface and a catalytically active surface, and having a device for detecting the difference in temperature between the two surfaces in order to determine the gas concentration in the surroundings of the sensor on the basis of the detected difference in temperature, wherein the catalytically active surface is located on the one side and the catalytically inactive surface is located on the opposite side of the sensor element, and the two surfaces are connected to one another via a thermal element, and wherein the sensor comprises a device for measuring the thermoelectric voltage between the catalytically active surface and the inactive surface of the sensor element as a measure of the difference in temperature and therefore the gas concentration, **characterized in that** the sensor element (1) comprises a hot plate (11) which is mounted on a base carrier (8) by means of narrow arms (7), on the front side of which hot plate (11) the catalytically active surface (3) is formed, and on the rear side of which the catalytically inactive surface (4) is formed, or vice versa, and **in that** the thermal element is formed by at least one via (2, 9) which extends through the hot plate (11) and connects the two surfaces (3, 4) to one another, and in the region of which the thermoelectric voltage is measured.

2. Sensor according to Claim 1, **characterized in that** the at least one via (2) comprises a material A, **in that** the two sides of the via (2) are in contact via a material B with a comparison point at which there is no temperature gradient present, and **in that** the material B produces a conductive connector as far as the connection for measuring the voltage.

3. Sensor according to Claim 2, **characterized in that** the voltage is tapped at the two feed lines (6) made of the material B.

4. Sensor according to one of the preceding claims, **characterized in that** it only has two connections to the sensor element (1).

5. Sensor according to one of the preceding claims, **characterized in that** the material A of the at least one via (2) is a material with a Seebeck coefficient which is high in terms of absolute value.

6. Sensor according to one of Claims 2 to 5, **characterized in that** the comparison point is located at a frame (8) of the hot plate (11).

7. Sensor according to one of the preceding claims, **characterized in that** it comprises a series circuit of thermal pairs over the surface of the sensor element (1).

8. Sensor according to Claim 7, **characterized in that** it has a plurality of vias (2, 9) which are connected in series and are made of different materials in the sensor element (1).

9. Sensor according to Claim 8, **characterized in that** the vias (2, 9) which are connected in series are alternately composed of materials A and B, which have Seebeck coefficients which differ from one another.

10. Sensor according to one of the preceding claims, **characterized in that** the at least one via (2, 9) in the sensor element (1) is embodied as a ceramic conductor.

11. Sensor according to one of the preceding claims, **characterized in that** only the edge regions of the at least one via (2, 9) in the sensor element (1) are filled with a conductor.

## Revendications

1. Capteur de détection de gaz oxydables avec un élément de capteur, qui présente une face catalytiquement inactive et une face catalytiquement active, et avec un dispositif de détermination d'une différence de température entre les deux faces en vue de la détermination de la concentration en gaz dans l'environnement du capteur sur la base de la différence de température déterminée, dans lequel la face catalytiquement active se trouve sur un côté et la face catalytiquement inactive se trouve sur le côté opposé de l'élément de capteur et les deux faces sont reliées l'une à l'autre par un élément thermoélectrique et dans lequel le capteur comprend un dispositif de mesure de la tension thermoélectrique entre la face catalytiquement active et la face catalytiquement inactive de l'élément de capteur comme mesure de la différence de température et dès lors de la concentration en gaz, **caractérisé en ce que** l'élément de capteur (1) comprend une plaque chaude (11) posée par des bras étroits (7) sur un support de base (8), plaque dont le côté avant porte la face catalytiquement active (3) et dont la face arrière porte la face catalytiquement inactive (4) ou inversement, et **en ce que** l'élément thermoélectrique est formé par au moins un contact traversant (2, 9) s'étendant à travers la plaque chaude (11) et reliant les deux faces (3, 4) l'une à l'autre, dans la région duquel on mesure la tension thermoélectrique.

2. Capteur selon la revendication 1, **caractérisé en ce que** ledit au moins un contact traversant (2) comprend un matériau A, **en ce que** les deux côtés du contact traversant (2) sont en contact par un matériau B avec un point de comparaison, auquel il n'y a pas de gradient de température, et **en ce que** le matériau B établit une liaison conductrice jusqu'au raccord pour la mesure de la tension.

3. Capteur selon la revendication 2, **caractérisé en ce que** la prise de tension est effectuée sur les deux conducteurs d'alimentation (6) constitués du matériau B.

4. Capteur selon l'une quelconque des revendications précédentes, **caractérisé en ce qu'**il présente uniquement deux raccords à l'élément de capteur (1).

5. Capteur selon l'une quelconque des revendications précédentes, **caractérisé en ce que** le matériau A dudit au moins un contact traversant (2) est un matériau avec un coefficient de Seebeck d'une valeur élevée.

6. Capteur selon l'une quelconque des revendications 2 à 5, **caractérisé en ce que** le point de comparaison se trouve sur un cadre (8) de la plaque chaude (11).

7. Capteur selon l'une quelconque des revendications précédentes, **caractérisé en ce qu'**il comprend un montage en série d'éléments thermoélectriques sur la surface de l'élément de capteur (1).

8. Capteur selon la revendication 7, **caractérisé en ce qu'**il présente plusieurs contacts traversants connectés en série (2, 9) en des matériaux différents dans l'élément de capteur (1).

9. Capteur selon la revendication 8, **caractérisé en ce que** les contacts traversants (2, 9) connectés en série se composent alternativement des matériaux A et B, qui possèdent des coefficients de Seebeck différents l'un de l'autre.

10. Capteur selon l'une quelconque des revendications précédentes, **caractérisé en ce que** ledit au moins un contact traversant (2, 9) est réalisé dans l'élément de capteur (1) sous la forme d'un conducteur céramique.

11. Capteur selon l'une quelconque des revendications précédentes, **caractérisé en ce que** seules les régions de bord dudit au moins un contact traversant (2, 9) dans l'élément de capteur (1) sont remplies avec un conducteur.
